# EUROPÄISCHE PATENTSCHRIFT

(11) **EP 0 830 857 B1**
(45) Veröffentlichungstag und Bekanntmachung des Hinweises auf die Patenterteilung: **25.06.2003**
(21) Anmeldenummer: 97114542.0
(22) Anmeldetag: 22.08.1997
(51) Int. Cl.: A61K 7/50, A61K 7/48

(54) **Kosmetische Zubereitungen**
Cosmetic compositions
Compositions cosmétiques

(30) Priorität: 30.08.1996 DE 19635195
(43) Veröffentlichungstag der Anmeldung: 25.03.1998
(73) Patentinhaber: Cognis Deutschland GmbH & Co. KG, 40589 Düsseldorf (DE)
(72) Erfinder: Bigorra Llosas, Joaquim, Dr., 08203 Sabadell (ES); Pi Subirana, Rafael, Dr., 08400 Granollers (ES); Prat Queralt, Esther, Dr., 08238 Alella (ES)

(56) Entgegenhaltungen:
- EP-A- 0 564 656
- WO-A-94/16677
- DE-A- 4 309 568

## Beschreibung

### Gebiet der Erfindung

Die Erfindung betrifft kosmetische Zubereitungen, die man erhält, indem man Fettsäurealkanolaminester in Gegenwart von ausgewählten kosmetischen Ölen und gegebenenfalls Polyolen und/oder Emulgatoren quaterniert, ein Verfahren zu ihrer Herstellung sowie die Verwendung der Mischungen zur Herstellung von kosmetischen Zubereitungen.

### Stand der Technik

Die Quaternierung vom Alkanolaminestem, vorzugsweise Fettsäuretriethanolaminestern, wird üblicherweise mit niedermolekularen Alkoholen, insbesondere 2-Propanol, als Lösungsmittel durchgeführt. Nach der Quaternierung verbleibt der niedere Alkohol im allgemeinen im Produkt, die resultierenden kationischen Tenside vom Esterquattyp werden als 85 bis 90%ige Ware vermarktet. Durch den Alkoholzusatz wird eine fließfähige Konsistenz der Reaktionsmischung während der Quaternierung ebenso wie der erhaltenen Endprodukte garantiert. Die Verwendung niederer Alkohole ist jedoch wegen der niedrigen Flammpunkte problematisch. Hinzu kommt, daß bei Verwendung der Esterquats in hautkosmetischen Präparaten die Anwesenheit von Alkoholen unerwünscht ist, da sie wenig hautverträglich sind und eine entfettende Wirkung zeigen. Schließlich ist von Nachteil, daß die Mischungen Parfümöle nur sehr mangelhaft emulgieren und gelegentlich eine unbefriedigende Lagerstabilität aufweisen.

Die Aufgabe der vorliegenden Erfindung hat somit darin bestanden, ein Verfahren zur Herstellung von kosmetischen Zubereitungen auf Basis von Esterquats mit verbesserter hautkosmetischer Verträglichkeit und einer guten Lagerstabilität zu entwickeln.

### Beschreibung der Erfindung

Gegenstand der Erfindung sind kosmetische Zubereitungen, speziell Hautreinigungs- und -pflegemittel, die man erhält, indem man Fettsäurealkanolaminester in Gegenwart von kosmetischen Ölen mit einem Spreitwert im Bereich von 150 und 800 mm² in an sich bekannter Weise mit Alkylierungsmitteln quaterniert.

Es wurde nun überraschenderweise gefunden, daß sich die Quaternierung auch in Gegenwart der genannten kosmetischen Öle und gegebenenfalls Polyolen und/oder Emulgatoren durchführen läßt und auf diese Weise lösemittelfreie Zubereitungen auf Basis von Esterquats mit einer guten hautkosmetischen Verträglichkeit erhalten werden, die eine hohe Lagerstabilität aufweisen, d.h. die Produkte bleiben auch nach längerer Lagerung homogen und weisen eine konstante Viskosität auf. Des weiteren zeichnen sich die erfindungsgemäßen Zubereitungen unerwarteter Weise dadurch aus, daß sie Parfümöle in Emulsionen zu stabilisieren vermögen.

Ein weiterer Gegenstand der Erfindung betrifft ein Verfahren zur Herstellung von kosmetischen Zubereitungen, bei dem man Fettsäurealkanolaminester in Gegenwart von kosmetischen Ölen mit einem Spreitwert im Bereich von 150 und 800 mm² in an sich bekannter Weise mit Alkylierungsmitteln quatemiert.

### Esterquats und Fettsäuretrialkanolaminester

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE-C1 43 08 794** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen aber auch schwach polaren Ölkörpem (z.B. Dioctylether), durchführt. Bezüglich der Verwendung von Esterquats in der Haut- und Haarkosmetik sei auf die Übersicht von I.Shapiro in **Cosm.Toil. 109, 77 (1994)** verwiesen.

Fettsäuretriethanolaminester, die als Einsatzstoffe in Betracht kommen, folgen der Formel **(I),** 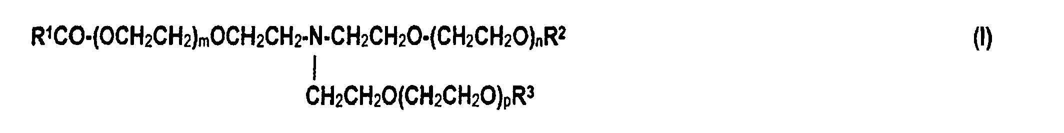 in der R¹CO für einen Acylrest mit 6 bis 22 Kohlenstoffatomen, R² und R³ unabhängig voneinander für Wasserstoff oder R¹CO, und m, n und p in Summe für 0 oder Zahlen von 1 bis 12 steht. Typische Beispiele für Fettsäuretriethanolaminester, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachin-säure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der resultierenden Esterquats hat sich ein Einsatzverhältnis von 1,2 : 1 bis 2,2 1, vorzugsweise 1,5 : 1 bis 1,9 : 1 als besonders vorteilhaft erwiesen. Die bevorzugten Fettsäuretriethanolaminester bzw. Esterquats stellen technische Mischungen von Mono-, Di- und Triestem mit einem durchschnittlichen Veresterungsgrad von 1,5 bis 1,9 dar und leiten sich von technischer C_{16/18}-Talg- bzw. Palmfettsäure (lodzahl 0 bis 40) ab. Aus anwendungstechnischer Sicht haben sich Fett-säuretriethanolaminester der Formel (I) als besonders vorteilhaft erwiesen, in der R¹CO für einen Acylrest mit 16 bis 18 Kohlenstoffatomen, R² für R¹CO, R³ für Wasserstoff, und m, n und p für 0 steht.

Neben den Fettsäuretriethanolaminestern kommen als Ausgangsstoffe ferner auch Ester von Fettsäuren mit Diethanolalkylaminen der Formel **(II)** in Betracht, 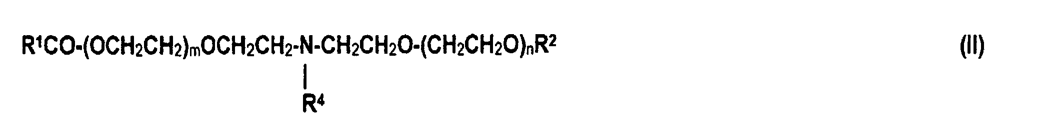 in der R¹CO für einen Acylrest mit 6 bis 22 Kohlenstoffatomen, R² für Wasserstoff oder R¹CO, R⁴ für Alkylreste mit 1 bis 4 Kohlenstoffatomen und m und n in Summe für 0 oder Zahlen von 1 bis 12 steht. Als weitere Gruppe geeigneter Einsatzstoffe für die Quatemierung sind schließlich die Ester von Fettsäuren mit 1,2-Dihydroxypropyldialkylaminen der Formel **(III)** zu nennen, 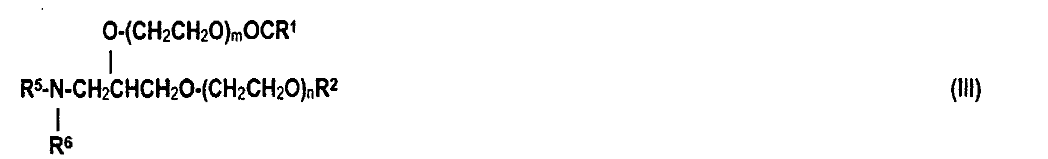 in der R¹CO für einen Acylrest mit 6 bis 22 Kohlenstoffatomen, R² für Wasserstoff oder R¹CO, R⁵ und R⁶ unabhängig voneinander für Alkylreste mit 1 bis 4 Kohlenstoffatomen und m und n in Summe für 0 oder Zahlen von 1 bis 12 steht. Hinsichtlich der Auswahl der bevorzugten Fettsäuren und des optimalen Veresterungsgrades gelten die für **(I)** genannten Beispiele auch für die Ester der Formeln **(II)** und **(III)**. Anstelle der reinen Fettsäuren können auch Mischungen mit Dicarbonsäuren wie beispielsweise Adipinsäure oder Dimerfettsäure im Mol-Verhältnis 1 : 5 bis 5 : 1, vorzugsweise 1 : 2 bis 2 : 1 eingesetzt werden.

### Kosmetische Öle

Unter kosmetischen Ölen im Sinne der vorliegenden Erfindung sind diejenigen Öle zu verstehen, die einen mittleren Spreitwert, d.h. einen Spreitwert im Bereich von 150 bis 800, bevorzugt von 200 bis 600 mm², aufweisen [vgl. S.Wallat et al. **Parf.Kosm. 11**, **768 (1994)].** Der Spreitwert ist ein Maß für die Spreitung des jeweiligen Öls auf der Haut. Unter Spreitung auf der Haut versteht man die Fähigkeit von Substanzen, sich auf der kapillaraktiven chemische heterogenen Homschicht mehr oder weniger schnell spontan auszubreiten und einen Film zu bilden. Mit der Spreitung eng verbunden sind Eigenschaften wie das Einziehvermögen in die Homschicht und der zeitliche Verlauf des Fettungsverhaltens **[Fette Seifen Anstrichmassen, 10, 403 (1985)].** Kosmetische Öle, die ein mittleres Spreitvermögen aufweisen, können z.B. Triglyceride, Guerbetalkohole oder Wachsester sein. Im Sinne der vorliegenden Erfindung eignen sich besonders Mandelöl, Paraffinöl (dick- und/oder dünnflüssig), Isopalmitinsäuretriglycerid, Oleylerucat, Oleyloleat, 2-Hexyldecylstearat, Decylerucat, Caprylsäuretriglycerid, Caprinsäuretriglycerid, 2-Octyldodecanol, Squalen, Cetylisononaoat, Sterylisononaoat, Oleylalkohol, Isocetylisopalmitat, Decyloleat, Octyldodecanol, 2-Hexyldecanol, Decansäuretetradecylester und/oder Isooctylstearat. Üblicherweise können die erfindungsgemäßen Zubereitungen die kosmetischen Öle in solchen Mengen enthalten, daß ihr Anteil 10 bis 90, vorzugsweise 50 bis 70 Gew.-% - bezogen auf die fertige Zubereitung - beträgt.

### Polyole

In einer bevorzugten Ausführungsform der Erfindung kann die Quaternierung zusätzlich in Gegenwart von Polyolen durchgeführt werden. Unter Polyolen sind Stoffe zu verstehen, die 2 bis 15 Kohlenstoffatome und mindestens zwei Hydroxylgruppen besitzen. Typische Beispiele sind:
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Die Polyole können in Mengen von 1 bis 50, insbesondere 5 bis 30 Gew.-% - bezogen auf die fertige Zubereitung - enthalten sein.

### Emulgatoren

In einer weiteren bevorzugten Ausführungsform der Erfindung kann die Quaternierung auch in Gegenwart von Emulgatoren durchgeführt werden. Als Emulgatoren kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574** und/oder Mischester von Fettsäuren mit 6 bis 22 Kohlenstoffatomen, Methylglucose und Polyolen, vorzugsweise Glycerin sowie
(b13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Vorzugsweise werden als Emulgatoren Fettalkoholpolyglycolether, Fettsäuremonoglyceridpolyglycolether, Polyglycerinpoly-12-hydroxystearate, Alkyloligoglucoside sowie Alkyloligoglucosid/Fettalkohol-Gemische.

Der Anteil der Emulgatoren kann 1 bis 30, vorzugsweise 5 bis 20 Gew.-% - bezogen auf die fertige Zubereitung - betragen. Praktisch bedeutet dies, daß die Alkylierung in Gegenwart mindestens eines kosmetischen Öls durchgeführt wird. Die Erfindung schließt jedoch die Möglichkeit ein, daß die Alkylierung auch in Gegenwart mindestens eines kosmetischen Öls und eines Polyols und/oder Emulgators erfolgt. Für die Einstellung eines gewünschten Gehaltes an kosmetischem Öl, Polyol und/oder Emulgator in der fertigen kosmetischen Zubereitung ist es erforderlich, die jeweils gewünschten Mengen dem Ester vor der Quaternierung zuzusetzen. Die erforderlichen Verhältnisse zu berechnen, bleibt dem Fachmann überlassen, der hierzu nicht erfinderisch tätig werden muß.

### Alkylierung

Die Alkylierung der Fettsäuretriethanolaminester kann in an sich bekannter Weise durchgeführt werden. Hierzu wird der Ester mit dem kosmetischen Öl, dem Polyol und/oder Emulgator in den berechneten Mengen vorgelegt und mit dem Alkylierungsmittel - das man üblicherweise in äquimolaren Mengen oder leichtem Unterschuß einsetzt - bei erhöhten Temperaturen (40 bis 95 °C) gerührt. Nach Abschluß der Reaktion kann nichtumgesetztes Alkylierungsmittel durch Zugabe einer geringen Menge Aminosäure, vorzugsweise Glycin, zerstört werden. Als Alkylierungsmittel kommen in diesem Zusammenhang Alkylhalogenide, Dialkylsulfate und Ethylenoxid - letzteres in Gegenwart von Dialkylphosphaten - in Betracht. Vorzugsweise betrifft das erfindungsgemäße Verfahren die Herstellung methylquatemierter Esterquats in Form ihrer Chloride oder Methylsulfat-Salze sowie Esterquat-Salze, die mit 1 bis 5 Mol Ethylenoxid quatemiert worden sind.

### Gewerbliche Anwendbarkeit

Die erfindungsgemäßen Mischungen sind lagerstabil, zeichnen sich durch eine besondere dermatologische Verträglichkeit aus und verleihen der Haut ein langanhaltendes Glättegefühl. Ein weiterer Gegenstand der Erfindung betrifft daher ihre Verwendung der Mischungen zur Herstellung von kosmetischen Zubereitungen, bei denen es sich in erster Linie um Hautreinigungs- und -pflegemittel wie beispielsweise Cremes, Lotionen, Duschbädern, Schaumbädern und dergleichen handelt. Darüber hinaus können sie auch in haarkosmetischen Mitteln, wie beispielsweise Shampoos und Conditionern eingesetzt werden. Als weitere Hilfs- und Zusatzstoffe können die Zubereitungen Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe enthalten.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte VinylpyrrolidonNinylimidazol-Polymere wie z.B. Luviquat® (BASF AG, Ludwigshafenl FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quatemierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

### Beispiele 1 und 2, Vergleichsbeispiel V1

In einem 1-l-Dreihalskolben mit Rührer, Innenthermometer und Destillationsaufsatz wurden 513 g (1,9 mol) teilgehärtete C_{16/18}-Talgfettsäure (lodzahl = 40), 149 g (1 mol) Triethanolamin und 1,4 g 50 Gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Talgfettsäuretriethanolaminester abgekühlt, der Reaktionsansatz entspannt und 0,4 g Wasserstoffperoxid zugegeben. Anschließend wurde in einem 500-ml-Dreihalskolben mit Rührer, Tropftrichter und Rückflußkühler eine Mischung von 45 g (0,072 mol) des Esters in 105 g kosmetischem Öl, [**1** = Mandelöl, **2** = Octyldodecanol, (erfindungsgemäße Beispiele), **3** = Dioctylether (Vergleichsbeispiel)], sowie 60 g Glycerin, vorgelegt und unter Rühren auf 45°C erhitzt (Gewichtsverhältnis in der resultierenden Zubereitung Esterquat : kosmetisches Öl = 30 : 70). Innerhalb von 2 h wurden 8,5 g (0,067 mol) Dimethylsulfat zugetropft. Nach Beendigung der Zugabe wurde die Mischung weitere 2 h bei 60°C gerührt. Die Mischung wurde als hellfarbiges Öl in praktisch quantitativer Ausbeute erhalten.

### Anwendungstechnische Beurteilung

Jeweils 60 g der erfindungsgemäßen Mischungen 1 und 2 sowie der Vergleichsmischung V1 wurden unter langsamen Rühren bei Raumtemperatur in 40 ml Wasser emulgiert. Es wurden homogene, kosmetisch elegante Emulsionen erhalten, deren hautsensorische Beurteilung durch ein Panel bestehend aus 6 geschulten Probanden erfolgte. Dabei bedeutet (+) angenehmes, glattes Hautgefühl und (-) eher stumpfes Hautgefühl. Die Viskosität der Emulsionen wurde nach 1, 3 15 und 30 Tagen Lagerung bei 20 bzw. 40°C nach der Brookfield-Methode (RVT-Viskosimeter, 20°C, 10 Upm) bestimmt. Die Ergebnisse sind in Tabelle 1 zusammengefaßt.

**Tabelle 1**

| **Hautgefühl und Viskosität (Mengenangaben als Gew.-%)** | | | |
|---|---|---|---|
| | **Beispiel 1** | **Beispiel 2** | **Vergl.bsp. V1** |
| Esterquat | 21 | 21 | 21 |
| Mandelöl | 50 | - | - |
| Octyldodecanol | - | 50 | - |
| Dioctylether | - | - | 50 |
| Glycerin | 29 | 29 | 29 |
| ***Sensorische Bewertung*** | + | + | - |

| ***Viskosität [mPas]*** | | | |
|---|---|---|---|
| ***- 1 d, 20°C*** | 9.000 | 9.000 | 9.000 |
| ***- 3 d, 20°C*** | 8.950 | 8.950 | 9.300 |
| ***- 15 d, 20°C*** | 8.900 | 8.900 | 8.300 |
| ***- 30 d, 20°C*** | 8.900 | 8.800 | 7.050 |
| ***- 30 d, 40°C*** | 8.700 | 8.600 | 3.500 |

Man erkennt, daß stabile kosmetische Zubereitungen, die gleichzeitig ein vorteilhaftes Hautgefühl vermitteln, nur im Sinne der vorliegenden Erfindung erhalten werden.

## Patentansprüche

1. Kosmetische Zubereitungen, dadurch erhältlich, daß man Fettsäurealkanolaminester in Gegenwart von kosmetischen Ölen mit einem Spreitwert im Bereich von 150 und 800 mm² in an sich bekannter Weise mit Alkylierungsmitteln quaterniert.

2. Verfahren zur Herstellung von kosmetischen Zubereitungen nach Anspruch 1, **dadurch gekennzeichnet, daß** man Fettsäurealkanolaminester in Gegenwart von kosmetischen Ölen mit einem Spreitwert im Bereich von 150 und 800 mm² in an sich bekannter Weise mit Alkylierungsmitteln quaterniert.

3. Verfahren nach Anspruch 2, **dadurch gekennzeichnet, daß** man Fettsäurealkanolaminester der Formel **(I)** einsetzt, 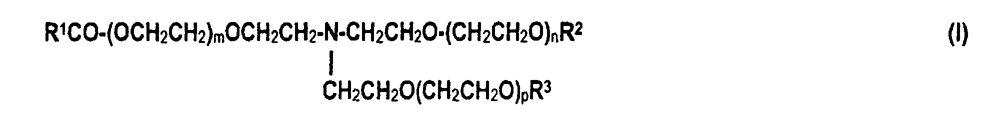 in der R¹CO für einen Acylrest mit 6 bis 22 Kohlenstoffatomen, R² und R³ unabhängig voneinander für Wasserstoff oder R¹CO, und m, n und p in Summe für 0 oder Zahlen von 1 bis 12 steht.

4. Verfahren nach den Ansprüchen 2 und 3, **dadurch gekennzeichnet, daß** man Fettsäurealkanolaminester der Formel **(II)** einsetzt, 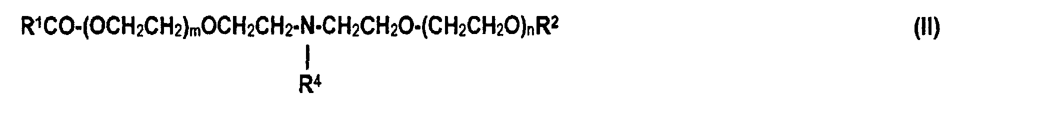 in der R¹CO für einen Acylrest mit 6 bis 22 Kohlenstoffatomen, R² für Wasserstoff oder R¹CO, R⁴ für Alkylreste mit 1 bis 4 Kohlenstoffatomen und m und n in Summe für 0 oder Zahlen von 1 bis 12 steht.

5. Verfahren nach den Ansprüchen 2 bis 4, **dadurch gekennzeichnet, daß** man Fettsäurealkanolaminester der Formel **(III)** einsetzt, 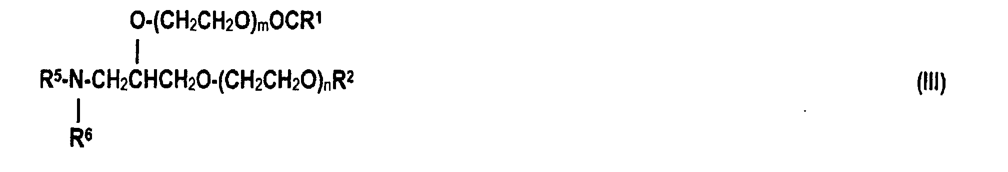 in der R¹CO für einen Acylrest mit 6 bis 22 Kohlenstoffatomen, R² für Wasserstoff oder R¹CO, R⁵ und R⁶ unabhängig von einander für Alkylreste mit 1 bis 4 Kohlenstoffatomen und m und n in Summe für 0 oder Zahlen von 1 bis 12 steht.

6. Verfahren nach den Ansprüchen 2 bis 5, **dadurch gekennzeichnet, daß** man als kosmetische Öle Guerbetalkohole, Wachsester und/oder Triglyceride einsetzt.

7. Verfahren nach den Ansprüchen 2 bis 6, **dadurch gekennzeichnet, daß** man die kosmetischen Öle in solchen Mengen einsetzt, daß ihr Anteil an den fertigen Zubereitungen 10 bis 90 Gew.-% beträgt.

8. Verfahren nach den Ansprüchen 2 bis 7, **dadurch gekennzeichnet, daß** man die Quaternierung in Gegenwart von Polyolen mit 2 bis 15 Kohlenstoffatomen und mindestens zwei Hydroxylgruppen durchführt.

9. Verfahren nach den Ansprüchen 2 bis 8, **dadurch gekennzeichnet, daß** man die Quaternierung in Gegenwart von Emulgatoren durchführt, die ausgewählt sind aus der Gruppe, die gebildet wird von Fettalkoholpolyglycolethern, Fettsäuremonoglyceridpolyglycolethern, Polyglycerinpoly-12-hydroxystearate, Alkyloligoglucoside und/oder Alkyloligoglucosid/Fettalkohol-Mischungen.

10. Verwendung der Mischungen nach den Anspruch 1 zur Herstellung von kosmetischen und/oder pharmazeutischen Zubereitungen.

## Claims

1. Cosmetic preparations obtainable by quaternizing fatty acid alkanolamine esters in known manner with alkylating agents in the presence of cosmetic oils having a spreading value of 150 to 800 mm².

2. A process for the production of the cosmetic preparations claimed in claim 1, **characterized in that** fatty acid alkanolamine esters are quaternized in known manner with alkylating agents in the presence of cosmetic oils having a spreading value of 150 to 800 mm².

3. A process as claimed in claim 2, **characterized in that** fatty acid alkanolamine esters corresponding to formula **(I):** 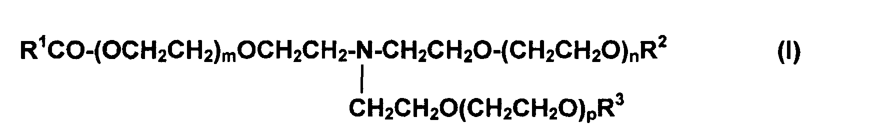 in which R¹CO is an acyl group containing 6 to 22 carbon atoms, R² and R³ independently of one another represent hydrogen or R¹CO and m, n and p together stand for 0 or for a number of 1 to 12, are used.

4. A process as claimed in claims 2 and 3, **characterized in that** fatty acid alkanolamine esters corresponding to formula **(II):** 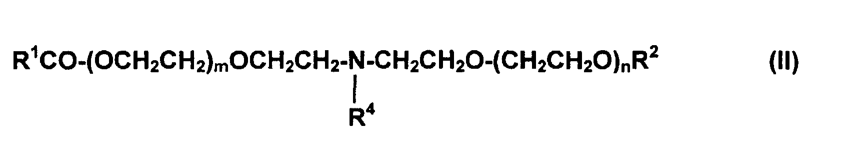 in which R¹CO is an acyl group containing 6 to 22 carbon atoms, R² is hydrogen or has the same meaning as R¹CO, R⁴ represents alkyl groups containing 1 to 4 carbon atoms and m and n together stand for 0 or for a number of 1 to 12,
are used.

5. A process as claimed in claims 2 to 4, **characterized in that** fatty acid alkanolamine esters corresponding to formula **(III):** 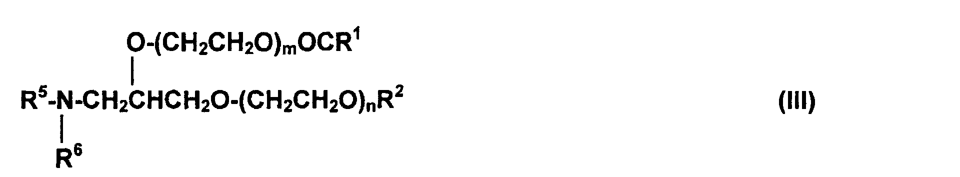 in which R¹CO is an acyl group containing 6 to 22 carbon atoms, R² is hydrogen or has the same meaning as R¹CO, R⁵ and R⁶ independently of one another represent alkyl groups containing 1 to 4 carbon atoms and m and n together stand for 0 or for a number of 1 to 12,
are used.

6. A process as claimed in claims 2 to 5, **characterized in that** Guerbet alcohols, wax esters and/or triglycerides are used as cosmetic oils.

7. A process as claimed in claims 2 to 6, **characterized in that** the cosmetic oils are used in such quantities that their percentage content in the final preparations is 10 to 90% by weight.

8. A process as claimed in claims 2 to 7, **characterized in that** the quaternization is carried out in the presence of polyols containing 2 to 15 carbon atoms and at least two hydroxyl groups.

9. A process as claimed in claims 2 to 8, **characterized in that** the quaternization is carried out in the presence of emulsifiers selected from the group consisting of fatty alcohol polyglycol ethers, fatty acid monoglyceride polyglycol ethers, polyglycerol-12-hydroxystearates, alkyl oligoglucosides and/or alkyl oligoglucoside/fatty alcohol mixtures.

10. The use of the mixtures claimed in claim 1 for the production of cosmetic and/or pharmaceutical preparations.

## Revendications

1. Préparations cosmétiques accessibles par le fait qu'on quaternise des esters d'alkanolamine d'acide gras en présence d'huiles cosmétiques ayant une valeur de dispersion dans la zone de 150 et 800 mm², avec des agents d'alkylation d'une manière connue en soi.

2. Procédé de production de préparations cosmétiques selon la revendication 1,
**caractérisé en ce qu'**
on quaternise d'une manière connue en soi des esters d'alkanolamine d'acide gras en présence d'huiles cosmétiques ayant une valeur de dispersion dans la zone de 150 et 800 mm², avec des agents d'alkylation.

3. Procédé selon la revendication 2,
**caractérisé en ce qu'**
on met en oeuvre des esters d'alkanolamine d'acide gras de formule (I) : 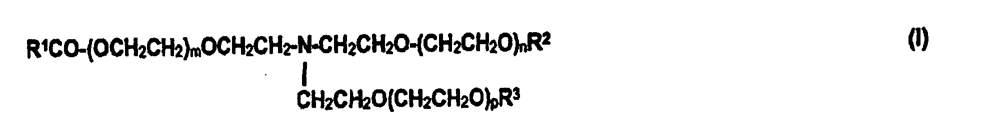 dans laquelle R¹CO représente un reste acyle ayant de 6 à 22 atomes de carbone, R² et R³ indépendamment l'un de l'autre représente de l'hydrogène ou R¹CO, et m, n et p globalement représentent 0, ou des nombres allant de 1 à 12.

4. Procédé selon les revendications 2 et 3,
**caractérisé en ce qu'**
on met en oeuvre des esters d'alkanolamine d'acide gras de formule (II) : 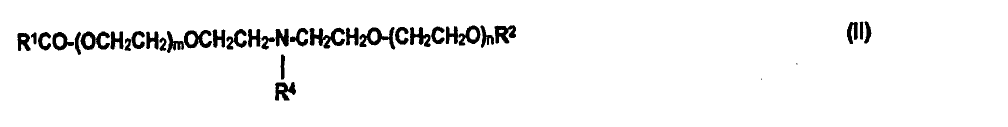 dans laquelle R¹CO représente un reste acyle, ayant de 6 à 22 atomes de carbone, R² représente de l'hydrogène ou R¹CO, R⁴ représente des restes alkyle ayant de 1 à 4 atomes de carbone et m et n globalement représentent 0 ou des nombres allant de 1 à 12.

5. Procédé selon les revendications 1 à 4,
**caractérisé en ce qu'**
on met en oeuvre des esters d'alkanolamines d'acide gras de formule (III) : 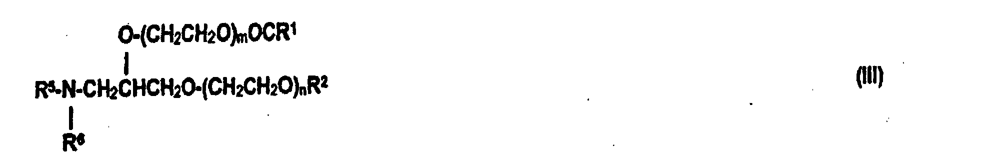 dans laquelle R¹CO représente un reste acyle ayant de 6 à 22 atomes de carbone, R² représente de l'hydrogène ou R¹CO, R⁵ et R⁶ indépendamment l'un de l'autre représentent des restes alkyle ayant de 1 à 4 atomes de carbone et m et n globalement représentent 0 ou des nombres allant de 1 à 12.

6. Procédé selon les revendications 2 à 5,
**caractérisé en ce qu'**
on met en oeuvre comme huiles cosmétiques des alcools de Guerbet, des esters de cire et/ou des triglycérides.

7. Procédé selon les revendications 2 à 6,
**caractérisé en ce qu'**
on met en oeuvre les huiles cosmétiques en quantité telle que leur proportion dans les préparations terminées est de 10 à 90 % en poids.

8. Procédé selon les revendications 2 à 7,
**caractérisé en ce qu'**
on effectue la quaternisation en présence de polyols ayant de 2 à 15 atomes de carbone et au moins deux groupes hydroxyle.

9. Procédé selon les revendications 2 à 8,
**caractérisé en ce qu'**
on effectue la quaternisation en présence d'agents émulsionnants qui sont choisis dans les groupe formé des polyglycoléthers d'alcools gras, des polyglycoléthers de monoglycéride d'acide gras et, des poly- 12-hydroxystéarates de polyglycényle, des alkyloligoglucosides et/ou des mélanges d'alkyloligoglucoside/alcool gras.

10. Utilisation des mélanges selon la revendication 1, en vue de la production de préparations cosmétiques et/ou pharmaceutiques.
